(19) Europäisches Patentamt
European Patent Office
Office européen des brevets

(11) Veröffentlichungsnummer : **0 137 524**
**B1**

(12) # EUROPÄISCHE PATENTSCHRIFT

(45) Veröffentlichungstag der Patentschrift : **20.01.88**

(51) Int. Cl.⁴ : **A 61 K 7/13**

(21) Anmeldenummer : **84200968.0**

(22) Anmeldetag : **04.07.84**

(54) **Mittel zum Färben von Haaren.**

(30) Priorität : **21.09.83 DE 3334030**

(43) Veröffentlichungstag der Anmeldung : **17.04.85 Patentblatt 85/16**

(45) Bekanntmachung des Hinweises auf die Patenterteilung : **20.01.88 Patentblatt 88/03**

(84) Benannte Vertragsstaaten : **CH DE FR GB IT LI**

(56) Entgegenhaltungen :
**EP-B- 0 008 079**
**DD-A- 57 402**
**DE-A- 1 949 750**
**DE-B- 1 142 045**
**FR-A- 1 397 551**
**FR-A- 1 398 193**
**FR-A- 1 401 469**
**Kirk-Othmer, Encyclopedia of Chemical Technology, Bd. 12 (3.Aufl.1978), S. 101-104**

(73) Patentinhaber : **Rütgerswerke Aktiengesellschaft**
**Mainzer Landstrasse 217**
**D-6000 Frankfurt a.Main 1 (DE)**

(72) Erfinder : **Orth, Winfried, Dr.**
**Am Schachtelgraben 28**
**D-6733 Hassloch/Pfalz (DE)**
Erfinder : **Schrader, Karl-Heinz, Dr.**
**Erlengrund 16**
**D-3454 Bevern (DE)**
Erfinder : **Fickert, Werner, Dr.**
**Stockacher Strasse 14**
**D-6800 Mannheim 61 (DE)**

**EP 0 137 524 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Mittel zum Färben von Haaren auf der Basis von Oxidationsfarbstoffen in Kombination mit einschlägigen Entwicklern. Die Färbung der Haare erfolgt dabei durch Reaktion der Entwicklersubstanzen mit sogenannten Kupplersubstanzen oder Nuanceuren in alkalischem Medium in Gegenwart eines geeigneten Oxidationsmittels. Durch die im allgemeinen dabei entstehenden intensiven Farben mit sehr guten Echtheitseigenschaften und durch die große Variationsbreite der Farbtöne spielen diese Oxidationsfarben eine bedeutende Rolle in der Haarkosmetik.

Als Kuppler- oder Nuanceurkomponenten kennt man m-Phenylendiaminderivate, Phenole, Naphthole oder Resorcinderivate. Da diese Produkte alle toxikologisch und dermatologisch nicht unbedenklich sind, versucht man, auf die unbedenklichere Pyridinaminoverbindungen überzugehen.

So kennt man die Verwendung als Kupplersubstanz von
2,3 bzw. 2,5-Diaminopyridin aus DE-C-11 42 045
2,5-Diaminopyridin aus DD-A-57 402
Bis-aminopyridinen aus EP 0008079 B1
Dihydroxipyridinen aus DE-A-3 115 643
Hydroxi- und Alkoxipyridinaminen aus FR-A-1 397 551 und FR-A-1 398 193 oder
Pyridylaminobenzolen und Bispyridylaminen aus FR-A-1 401 469.

Aminopyridinverbindungen aber sind oxidationsempfindlich gegen Luftsauerstoff. Um Verluste, die bereits bei der Lagerung der Präparate sowie bei Gebrauch entstehen, auszugleichen, werden diese Verbindungen in größeren Mengen eindosiert als sie für den Färbvorgang an sich notwendig wären. Außerdem werden diese Aminoverbindungen als Salze eingesetzt, um somit eine Stabilisierung während der Lagerung zu erzielen. Dadurch aber handelt man sich andere Nachteile ein : Aufgrund der durch die Salzbildung bedingten höheren Anzahl an Ionen wird die Brillanz der Farbtöne gemindert. Außerdem sind diese Salze in höheren Konzentrationen nicht mit allen Tensiden kombinierbar, so daß nicht alle wünschenswerten galenischen Formen der Haarfärbemittel herstellbar sind.

Es bestand daher die Aufgabe, Haarfärbemittel auf Basis von Oxidationsfarbstoffen zu entwickeln, die intensive Farben mit sehr guten Echtheitseigenschaften in einer großen Variationsbreite der Farbtöne ergeben und deren Kuppler- oder Nuanceurkomponenten toxikologisch und dermatologisch unbedenklich und gegen Luftsauerstoff weitgehend beständig sind, so daß sie auch in geringen Mengen in neutraler oder Salzform eingesetzt werden können.

Es wurde gefunden, daß Nitropyridinderivate der allgemeinen Formel I

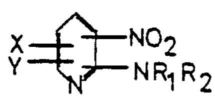

(I)

in der entweder $R_1$ Wasserstoff oder einen Alkylrest mit 1-4 C-Atomen und $R_2$ einen unsubstituierten oder an beliebiger Stelle mit einer oder mehreren Amino-, Methylamino-, Dimethylamino-, Hydroxi-, Alkyl- oder Alkoxigruppen mit 1-2 C-Atomen substituierten Phenylrest, einen Cycloalkylrest mit bis zu 7 C-Atomen oder einen unsubstituierten oder an beliebiger Stelle methyl-, äthyl- oder propylsubstituierten Pyrrol-, Pyridin-, Piperidin-, Pyrimidin-, Piperazin- oder Morpholinrest darstellen oder $R_1$ und $R_2$ gleich oder verschieden sind und Wasserstoff, einen Alkylrest mit 1-4 C-Atomen, oder einen Rest der allgemeinen Formel II

$$—R—Z \quad (II)$$

darstellen, wobei R eine Alkylenkette von 1-6 C-Atomen oder eine Phenylengruppe und Z eine an beliebiger Stelle stehende Hydroxyl- und/oder eine Alkoxigruppe mit 1-3 C-Atomen oder eine Aminogruppe der allgemeinen Formel III

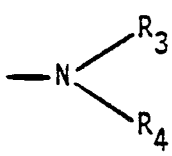

(III)

darstellt, in der $R_3$ und $R_4$ gleich oder verschieden sind und Wasserstoff oder einen unsubstituierten oder an beliebiger Stelle hydroxyl- oder aminsubstituierten Alkylrest mit bis zu 7 C-Atomen darstellen oder $R_3$ Wasserstoff oder einen unsubstituierten oder an beliebiger Stelle hydroxyl- oder aminsubstituierten Alkylrest mit bis zu 7 C-Atomen und $R_4$ einen unsubstituierten oder an beliebiger Stelle mit einer oder mehreren Amino-, Methylamino-, Dimethylamino-, Hydroxi-, Alkyl- oder Alkoxigruppen mit 1-2 C-Atomen substituierten Phenylrest oder einen unsubstituierten oder an beliebiger Stelle methyl-, äthyl- oder propylsubstituierten Pyrrol-, Pyridin-, Piperidin-, Pyrimidin-, Piperazin- oder Morpholinrest bedeuten und

X und Y gleich oder verschieden sind und jeweils entweder Wasserstoff, eine Alkyl- oder Alkoxigruppe mit 1-3 C-Atomen oder eine Aminogruppe der allgemeinen Formel III darstellen, in Kombination mit einschlägigen Entwicklerkomponenten und Oxidationsmitteln ideale Kuppler- oder Nuanceurkomponenten darstellen, die die oben genannten Anforderungen erfüllen.

Weitere geeignete Nuanceurkomponenten sind 2-Morpholino-5-nitropyridin und 2-Benzylamino-5-nitropyridin.

Es ist allgemein bekannt, daß die Nitrogruppe die Oxidations-beständigkeit von Pyridinderivaten steigert. Diese Kenntnis hat denn auch bislang den Einsatz von Nitropyridinderivaten in Färbemitteln auf Basis von Oxidationsfarbstoffen verhindert, denn die dort eingesetzten Produkte müssen ja mit einem gängigen Oxidationsmittel leicht reagieren. Daher sind bestimmte Nitropyrindinderivate zwar aus DE-PS 19 49 750 als selbstaufziehende Haarfarben bekannt, nicht aber ihre Verwendung in Färbemitteln auf Basis von Oxidationsfarben. Oberraschenderweise sind die Nitropyridinderivate gemäß der Erfindung zwar gegen Luftsauerstoff ausreichend stabil, daß sie auch in ihrer nichtionogenen Form eingesetzt werden können, aber trotzdem so reaktiv, daß sie mit gängigen Oxidationsmitteln in der an sich bekannten Weise im Sinne der Färbung reagieren.

Es wurde weiterhin gefunden, daß die Nitropyridinderivate gemäß der Erfindung sowohl toxikologisch als auch dermatologisch als unbedenklich angesehen werden können. Dies ist insofern überraschend, als aus der Aromatenchemie bekannt ist, daß die Nitroprodukte allgemein eine höhere Toxizität zeigen als die nicht nitrierten Aromaten.

Weitere Vorteile der erfindungsgemäßen Haarfärbemittel sind äußerst brilante Farbtöne, die sich bei Verwendung der als Kupplersubstanzen bekannten Aminopyridine nicht erzielen lassen, sowie eine stärkere Farbgebung trotz niedriger Einsatzmengen. Dieser letzte Effekt ist teils bedingt durch die farbgebende Wirkung der Nitrogruppe und teils durch die Oxidationsstabilität. Dadurch wird es ermöglicht, mit etwa der halben Menge der erfindungsgemäßen Nitropyridinderivate anstelle der bislang verwendeten Aminopyridinderivate gleich tiefe, aber brillantere Farbtöne zu erzielen.

Durch Variation der Substitutenten am Pyridinring und an der Aminogruppe der erfindungsgemäß eingesetzten Nitropyridinverbindungen lassen sich verschiedene Farbvarianten erzielen. Es ist somit mit diesen Mitteln möglich, durch Abmischung verschiedener Nuanceurkomponenten nahezu alle Farbvarianten mit einem Oxidationsfarbensystem einzustellen. Die erfindungsgemäßen Haarfärbemittel stellen somit eine Bereicherung auf dem Gebiet der Haarkosmetik dar.

Erfindungsgemäß in Oxidationsfarbstoffsystemen einzusetzende Kuppler- und Nuanceurkomponenten sind theoretisch alle Pyridinderivate, die eine Nitrogruppe und mindestens eine substituierte oder unsubstituierte Aminogruppe in beliebiger Stellung am Pyridinring enthalten. Verkaufsfähige Produkte sollen jedoch in Wasser ggf. als Salz oder im Gemisch mit einem Lösungsvermittler löslich und stabil sein, so daß von daher gewisse Einschränkungen bezüglich der Substituenten vorgegeben sind. In der Praxis werden daher die Nitropyridinderivate verwendet, die der allgemeinen Formel (I) entsprechen

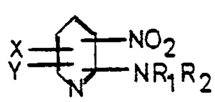

(I)

Hierbei können wahlweise alle Substituenten am Pyridinring jeweils wechselseitig in 2-, 3-, 4-, 5- oder 6-Stellung vorhanden sein, wobei dann jeweils die anderen Substituenten beliebige andere Positionen einnehmen können. Die Substituenten haben die folgenden Bedeutungen :

X und Y können gleich oder verschieden sein und entweder Wasserstoff, eine Alkyl-, Hydroxyalkyl-, oder Alkoxigruppe mit 1-3 C-Atomen oder eine Aminogruppe sein. Beispiele für derartige Substituenten sind die folgenden Gruppen : Methyl-, Äthyl-, Propyl-, i-Propyl-, Hydroximethyl-, Hydroxyäthyl-, Hydroxipropyl-, Methoxi-, Äthoxi- oder Propoxi-. Die Aminogruppe entspricht der allgemeinen Formel III

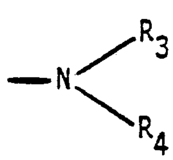

(III)

in der $R_3$ und $R_4$ gleich oder verschieden sind und Wasserstoff oder einen unsubstituierten oder an beliebiger Stelle hydroxyl- oder aminsubstituierten Alkyl-, Aralkyl-, Cycloalkylrest mit bis zu 7 C-Atomen einen unsubstituierten oder an beliebiger Stelle mit einer oder mehreren Amino-, Methylamino-, Dimethylamino-, Hydroxi-, Alkyl- oder Alkoxigruppe mit 1-2 C-Atomen substituierten Phenylrest oder einen unsubstituierten oder an beliebiger Stelle methyl-, äthyl- oder propylsubstituierten Pyrrol-, Pyridin-, Piperidin-, Pyrimidin-, Piperazin- oder Morpholinrest bedeuten.

Beispiele für diese Aminogruppe sind die unsubstituierte Aminogruppe selbst sowie die Methyl-, Dimethyl-, Äthyl-, Diäthyl-, Methyläthyl-, Propyl-, i-Propyl-, Dipropyl-, Diisopropyl-, Methylpropyl-, Methyl-i-propyl-, Äthylpropyl-, Butyl-, i-Butyl-, tert. Butyl-, Aminomethyl-, Aminoäthyl-, Aminopropyl-, Aminobutyl-, Aminopentyl-, Aminohexyl-, Aminocyclohexyl-, Bis-aminomethyl-, Bis-aminoäthyl-, Bis-aminopropyl-, Bis-aminobutyl-, Bis-aminopentyl-, Bis-aminohexyl-, Hydroximethyl-, Dihydroximethyl-, Hydroxiäthyl-, Dihydroxiäthyl-, Hydroxipropyl-, Dihydroxipropyl-, Phenyl-, Aminophenyl-, Hydroxiphenyl-, Methoxiphenyl-, Tolyl- oder Äthylphenylgruppe.

Der Substituent $—NR_1R_2$ kann eine unsubstituierte oder substituierte Aminogruppe sein, wobei $R_1$ Wasserstoff oder einen Alkylrest mit 1-4 C-Atomen und $R_2$ einen unsubstituierten oder an beliebiger Stelle mit einer oder mehreren Amino-, Methylamino-, Dimethylamino-, Hydroxi-, Alkyl- oder Alkoxigruppen mit 1-2 C-Atomen substituierten Phenylrest, einen Cycloalkylrest mit bis zu 7 C-Atomen oder einen unsubstituierten oder an beliebiger Stelle methyl-, äthyl- oder propylsubstituierten Pyrrol-, Pyridin-, Piperidin-, Pyrimidin-, Piperazin- oder Morpholinrest darstellen oder $R_1$ und $R_2$ gleich oder verschieden sind und Wasserstoff oder einen Alkylrest mit 1-4 C-Atomen darstellen.

Beispiele für diese Aminogruppe sind die Methyl-, Äthyl-, Propyl-, i-Propyl-, Butyl-, i-Butyl-, tert. Butyl-, Dimethyl-, Diäthyl-, Methyl-äthyl-, Dipropyl, Di-i-propyl-, Methylpropyl-, Cyclohexyl-, Phenyl-, Aminophenyl-, Diaminophenyl-, Methylaminophenyl-, Dimethylaminophenyl-, Hydroxiphenyl-, Dihydroxiphenyl-, Tolyl-, Xylyl-, Äthylphenyl-, Methoxiphenyl-, Äthoxiphenyl- oder Dimethoxiphenylaminogruppe.

Die Substituenten $R_1$ und $R_2$ können aber auch einen Rest der allgemeinen Formel

$$—R—Z$$

darstellen, wobei R eine Alkylenkette mit 1-6 C-Atomen oder eine Phenylengruppe und Z eine an beliebiger Stelle stehende Hydroxyl- und/oder Alkoxigruppe mit 1-3 C-Atomen oder eine Aminogruppe der allgemeinen Formel

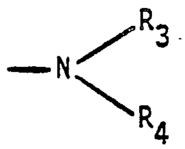

bedeuten.

$R_3$ und $R_4$ haben die im vorangegangenen Text angegebene Bedeutung. Beispiele für derartige Aminogruppen sind die

Hydroximethyl-, Hydroxiäthyl-, Hydroxipropyl-, Hydroisopropyl-, Hydroxibutyl-, Hydroxipentyl-, Hydroxihexyl-, Methoximethyl-, Äthoximethyl-, Propoximethyl-, Isopropoximethyl-, Hydroxymethoximethyl-, Hydroxiäthoxymethyl-, Methoxiäthyl-, Äthoxyäthyl-, Aminomethyl-, Aminoäthyl-, Aminopropyl-, Aminobutyl-, Aminopentyl-, Aminohexyl-, Aminocyclohexyl-, Methylaminomethyl-, Methylaminoäthyl-, Methylaminopropyl-, Dimethylaminomethyl-, Dimethylaminoäthyl-, Dimethylaminopropyl-, Äthylaminomethyl-, Propylaminomethyl-, Diäthylaminomethyl-, Äthylaminoäthyl-, Diäthylaminoäthyl-, Äthylaminopropyl-, Diäthylaminopropyl-, Bis-aminomethyl-, Bis-aminoäthyl-, Bis-aminopropyl-, Bis-aminobutyl-, Bis-aminopentyl-, Bis-aminohexyl-, Hydroxiphenyl-, Methoxiphenyl-, Tolyl-, Dimethoxiphenyl-, Aminophenyl-, Phenyl-, Diaminophenyl-, Pyrrolmethyl-, Pyrroläthyl-, Methylpyrrolmethyl-, Methylpyrroläthyl-, Pyridinmethyl-, Pyridinäthyl-, Pyridinpropyl-, Methylpyridinmethyl-, Methylpyridinäthyl-, Dimethylpyridinmethyl-, Dimethylpyridinäthyl-, Äthylpyridinmethyl-, Äthylpyridinäthyl-, Piperidinmethyl-, Piperidinäthyl-, Methylpiperidinmethyl-, Äthylpiperidinäthyl-, Piperazinmethyl-, Piperazinäthyl-, Methylpiperazinmethyl-, Methylpiperazinäthyl-, Äthylpiperazinmethyl-, Äthylpiperazinäthyl-, Pyrimidinmethyl-, Pyrimidinäthyl-, Pyrimidinpropyl-, Methylpyrimidinmethyl-, Morpholinmethyl-, Methylmorpholinmethyl-, Äthylmorpholinmethyl-, Morpholinäthyl-, Methylmorpholinäthyl-, Äthylmorpholinäthyl-, Propylpyrrolmethyl-, Propylpyridinmethyl-, Propylpiperinmethyl-, Propylpyrimidinmethyl-, Propylpiperazinmethyl-o. Propylmorpholinmethylamin.

Die erfindungsgemäßen Nuanceurkomponenten können jeweils alleine oder zur Einstellung gewünschter Farbnuancen in Abmischung miteinander oder mit anderen an sich bekannten Nuanceur- oder Kupplerkomponenten eingesetzt werden.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie

p-Phenylendiamin, Alkylamino-p-phenylendiamine
p-Toluylendiamin,
p-Aminophenol,
N-Methyl-p-phenylendiamin,
N,N-Dimethyl-p-phenylendiamin,
N,N-Diäthyl-2-methyl-p-phenylendiamin,
N-Äthyl-N-hydroxyäthyl-p-phenylendiamin,
Chlor-p-phenylendiamin,

N,N-Bis-hydroxyäthylamino-p-phenylendiamin,
Methoxy-p-phenylendiamin,
2,6-Dichlor-p-phenylendiamin,
2-Chlor-6-brom-p-phenylendiamin,
2-Chlor-6-methyl-p-phenylendiamin,
6-Methoxy-3-methyl-p-phenylendiamin,

andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner heterocyclische Hydrazonderivate wie

1-Methylpyrrolidon-(2)-hydrazon,

4-Aminopyrazolonderivate wie

4-Amino-1-phenyl-3-carbamoylpyrazolon-5,
N-Butyl-N-sulfobutyl-p-phenylendiamin,

Tetraaminopyrimidine wie

2,4,5,6-Tetraaminopyrimidin,
4,5 Diamino-2,6-bismethylaminopyrimidin,
2,5-Diamino-4-diäthylamino-6-methylaminopyrimidin,
2,4,5-Triamino-6-dimethylaminopyrimidin,
2,4,5-Triamino-6-piperidino-pyrimidin,
2,4,5-Triamino-6-anilino-pyrimidin,
2,4,5-Triamino-6-morpholinopyrimidin,
2,4,5-Triamino-6-β-hydroxy-äthylaminopyrimidin

aber auch Pyridinderivate wie z. B. 2,5-Diaminopyridin oder 2,5-Diamino-4-methylpyridin anzuführen.

Die oxidative Kupplung, d. h. die Entwicklung der Färbung könnte grundsätzlich, wie bei anderen Oxidationshaarfarbstoffen auch durch Luftsauerstoff erfolgen. Jedoch ist für die praktische Anwendung die Reaktionsgeschwindigkeit zu gering und die Farbentwicklung auf dem Haar zu langsam. Daher werden bevorzugt chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungs-verbindungen mit Kaliumperoxidisulfat in Betracht.

Die erfindungsgemäßen Haarfärbemittel, die die Nuanceur- und Entwicklerkomponente enthalten, werden für den Einsatz in entsprechende kosmetische Zubereitungen wie Cremes, Emulsionen, Gele oder auch einfach Lösungen eingearbeitet. Dazu ist es mitunter notwendig, die Lösungen bis zu 100 °C zu erhitzen, um die Komponenten in Lösung zu bringen, ggf. unter Mithilfe eines Lösungsvermittlers. Dabei liegen in gebrauchsfähigen Produkten die Konzentrationen an Nuanceurkomponenten zwischen 0,01-2 Gewichtsprozent und an Entwicklerkomponente zwischen 0,1-5 Gewichtsprozent. Zur Herstellung der kosmetischen Zubereitungen werden die Komponenten mit den für derartige Zubereitungen üblichen weiteren Bestandteilen gemischt. Als solche zusätzlichen Bestandteile sind zum Beispiel Ammoniumhydroxid, Netz- oder Emulgiermittel vom anionischen oder nichtionogenen Typ wie Alkylbenzolsulfonate, Fettalkoholsulfonate, Fettalkoholäthersulfate, Aminoxide, Alkylsulfonate, Fettsäurealkanolamide, Alkylphenoloxathylate und Anlagerungsprodukte von Äthylenoxid an Fettalkohole, Reduktionsmitteln, wie Natriumsulfit, Natriumdithionit, Thioglykolsäure oder Ascorbinsäure, Verdickungsmittel wie Methylcellulose, höhere Fettalkohole, Fettsäuren, ferner Parfümöle und Haarpflegemittel wie Pantothensäure und Cholesterin zu nennen.

Kurz vor der Anwendung werden diese Haarfärbemittel mit einer Lösung eines der genannten Oxidationsmittel wie üblich gemischt und die so erhaltene Mischung auf das Haar aufgetragen. Die Anwendungstemperaturen bewegen sich dabei im Bereich von 30 bis 40 °C. Nach einer Einwirkungsdauer von ca. 30 Minuten wird das Haarfärbemittel vom zu färbenden Haar durch Spülen entfernt. Hernach wird das Haar mit einem milden Schampoo nachgewaschen und getrocknet.

## Beispiele

mit Haarfärbemitteln folgender Grundzusammensetzung werden Färbeversuche durchgeführt :

2 Gew.-% 30 %ige Fettsäureaminoxidlösung
0,5 Gew.-% Natriumdithionit
10 Gew.-% 25 %iges Ammoniumhydroxid
0,5 Gew.% erfindungsgemäßer Nuanceurkomponente
1 Gew.-% Entwicklerkomponente

86 Gew.-% Wasser

10 ml der Haarfärbemittel werden mit 10 ml Wasserstoffperoxidlösung (6 %) gemischt. In diese Mischungen werden Haarsträhnen aus Naturhaar eingetaucht und die Farblösung läßt man 30 Minuten bei 35 °C einwirken. Anschließend werden die Strähnen gut ausgespült, getrocknet und hinsichtlich ihrer Färbung beurteilt.
Verwendete Entwicklerkomponenten :

E1 : p-Toluylendiamin
E2 : 2,5-Diaminopyridin
E3 : 2,5-Diamino-4-methylpyridin

Verwendete erfindungsgemäße Nuanceurkomponenten :

N1 : 3-Amino-2-nitropyridin
N2 : 2-(2'-Aminoäthylamino)-3-nitropyridin
N3 : 2-Amino-3-nitropyridin
N4 : 2-(2'-Hydroxiäthylamino)-5-nitropyridin
N6 : 2-(2'-Aminoäthylamino)-5-nitropyridin
N7 : 2-(3'-Dimethylaminopropylamino)-5-nitropyridin
N8 : 2-(2'-Aminöathylamino)-6-methoxi-3-nitropyridin
N9 : 2-(2'-Hydroxiphenylamino)-6-methoxi-3-nitropyridin
N10 : 2-(2'-Hydroxiäthylamino)-6-methoxi-3-nitropyridin
N11 : 2-(2'-Methoxiphenylamino)-6-methoxi-3-nitropyridin
N12 : 2-(4'-Hydroxiphenylamino)-6-methoxi-3-nitropyridin
N13 : 2-(N-Methyl-2-hydroxiäthylamino)-5-nitropyridin
N14 : 2-(4'-Amino-5'-methylphenylamino)-6-methoxi-3-nitropyridin
N15 : 2-(4'-Hydroxiphenylamino)-5-nitropyridin
N17 : 2-(n-Propylamino)-5-nitropyridin
N18 : 2-(2'-Hydroxiäthylaminoäthylamino)-5-nitropyridin
N19 : 2-Amino-4-methyl-5-nitropyridin
N20 : 2-Amino-4-methyl-3-nitropyridin
N21 : 2-Amino-6-methyl-5-nitropyridin
N22 : 2,6-Diamino-3-nitropyridin
N23 : 2-(N-Dimethylaminoäthylamino)-5-nitropyridin
N24 : 2-Benzylamino-5-nitropyridin
N25 : 2-Cyclohexylamino-5-nitropyridin
N26 : 2-(1-Methyl-1H-pyrrol-2-äthylamino)-5-nitropyridin
N27 : 2-Morpholino-5-nitropyridin
N28 : 6-Ethoxi-2-methylamino-3-nitropyridin
N29 : 2,6-Bis-(2-hydroxiäthylamino)-3-nitropyridin

Die erhaltenen Färbungen sind nachstehender Tabelle 1 zu entnehmen.

Tabelle 1

| Beispiel | Entwickler | Nuanceur | Farbeffekt |
|---|---|---|---|
| 1 | E 1 | N 1 | Blondton mit leichtem Grünstich |
| 2 | E 1 | N 2 | warmer Blondton |
| 3 | E 2 | N 2 | rotbraun |
| 4 | E 3 | N 2 | gelbbraun |
| 5 | - | N 3 | kein Effekt |
| 6 | E 1 | N 3 | mittelblond |
| 7 | E 2 | N 3 | schwach orange |
| 8 | E 3 | N 3 | schwach gelb |
| 9 | E 1 | N 4 | mittelblond |
| 10 | E 2 | N 4 | hell gelborange |
| 11 | E 3 | N 4 | hell gelbbraun |
| 12 | E 1 | N 6 | kastanienbraun |

| Beispiel | Entwickler | Nuanceur | Farbeffekt |
|---|---|---|---|
| 13 | E 2 | N 6 | gelborange |
| 14 | E 3 | N 6 | gelbbraun |
| 15 | E 1 | N 7 | dunkel rotbraun |
| 16 | E 2 | N 7 | gelborange |
| 17 | E 3 | N 7 | gelbbraun |
| 18 | E 1 | N 8 | blondgrün |
| 19 | E 2 | N 8 | hell orangebraun |
| 20 | E 3 | N 8 | gelborange |
| 21 | - | N 9 | kein Effekt |
| 22 | E 1 | N 9 | rotbraun |
| 23 | E 2 | N 10 | mahagoni |
| 24 | E 3 | N 10 | goldblond |
| 25 | E 2 | N 11 | kastanienbraun |
| 26 | E 3 | N 11 | goldblond |
| 27 | E 1 | N 12 | violett-braun |
| 28 | E 1 | N 13 | dunkelbraun |
| 29 | E 1 | N 14 | rotbraun |
| 30 | E 1 | N 15 | orange-rotbraun |
| 31 | - | N 16 | kein Effekt |
| 32 | E 1 | N 17 | schwarzbraun |
| 33 | E 1 | N 18 | braun |
| 34 | E 1 | N 19 | kastanie |
| 35 | E 1 | N 20 | rotbraun |
| 36 | E 1 | N 21 | dunkelbraun |
| 37 | E 1 | N 22 | kastanienbraun |
| 38 | E 1 | N 23 | kastanienbraun |
| 39 | E 1 | N 24 | kastanienbraun |
| 40 | - | N 25 | orange |
| 41 | E 1 | N 23 | rostbraun |
| 42 | - | N 26 | rotorange |
| 43 | E 1 | N 26 | mittelbraun |
| 44 | E 1 | N 27 | hellbraun |
| 45 | E 1 | N 28 | blau |
| 46 | E 1 | N 29 | blond |

**Patentansprüche**

1. Mittel zum Färben von Haaren auf der Basis von Oxidationsfarbstoffen in Kombination mit einschlägigen Entwicklern, dadurch gekennzeichnet, daß es als Nuanceurkomponente ein oder mehrere Nitropyridinderivate der allgemeinen Formel I enthält,

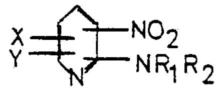

(I)

in der entweder $R_1$ Wasserstoff oder einen Alkylrest mit 1-4 C-Atomen und $R_2$ einen unsubstituierten oder an beliebiger Stelle mit einer oder mehreren Amino-, Methylamino-, Dimethylamino-, Hydroxi-, Alkyl- oder Alkoxigruppen mit 1-2 C-Atomen substituierten Phenylrest, einen Cycloalkylrest mit bis zu 7 C-Atomen oder einen unsubstituierten oder an beliebiger Stelle methyl-, äthyl- oder propylsubstituierten Pyrrol-, Pyridin-, Piperidin-, Pyrimidin-, Piperazin- oder Morpholinrest darstellen oder $R_1$ und $R_2$ gleich oder verschieden sind und Wasserstoff, einen Alkylrest mit 1-4 C-Atomen, oder einen Rest der allgemeinen Formel II

$$—R—Z \qquad (II)$$

darstellen, wobei R eine Alkylenkette von 1-6 C-Atomen oder eine Phenylengruppe und Z eine an beliebiger Stelle stehende Hydroxyl- und/oder eine Alkoxigruppe mit 1-3 C-Atomen oder eine Aminogruppe der allgemeinen Formel III

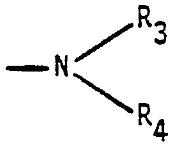

(III)

darstellt, in der $R_3$ und $R_4$ gleich oder verschieden sind und Wasserstoff oder einen unsubstituierten oder an beliebiger Stelle hydroxyl- oder aminsubstituierten Alkylrest mit bis zu 7 C-Atomen darstellen oder $R_3$ Wasserstoff oder einen unsubstituierten oder an beliebiger Stelle hydroxyl- oder aminsubstituierten Alkylrest mit bis zu 7 C-Atomen und $R_4$ einen unsubstituierten oder an beliebiger Stelle mit einer oder mehreren Amino-, Methylamino-, Dimethylamino-, Hydroxi-, Alkyl- oder Alkoxigruppen mit 1-2 C-Atomen substituierten Phenylrest oder einen unsubstituierten oder an beliebiger Stelle methyl-, äthyl- oder propylsubstituierten Pyrrol-, Pyridin-, Piperidin-, Pyrimidin-, Piperazin- oder Morpholinrest bedeuten und X und Y gleich oder verschieden sind und jeweils entweder Wasserstoff, eine Alkyl- oder Alkoxigruppe mit 1-3 C-Atomen oder eine Aminogruppe der allgemeinen Formel III darstellen.

2. Mittel zum Färben von Haaren auf der Basis von Oxidationsfarbstoffen in Kombination mit einschlägigen Entwicklern, dadurch gekennzeichnet, daß es als Nuanceurkomponente 2-Morpholino-5-nitropyridin enthält.

3. Mittel zum Färben von Haaren auf der Basis von Oxidationsfarbstoffen in Kombination mit einschlägigen Entwicklern, dadurch gekennzeichnet, daß es als Nuanceurkomponente 2-Benzylamino-5-nitropyridin enthält.

**Claims**

1. A composition for the dyeing of hair on the basis of oxidation dyestuffs in combination with known developers characterized in that it contains as coupling agent at least one derivative of the general formula I

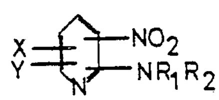

(I)

wherein either $R_1$ in hydrogen or an alkyl radical with 1 to 4 carbon atoms and $R_2$ is a phenyl radical which may be unsubstituted or substituted at any given position with one or more amino-, methylamino-, dimethylamino-, hydroxy-, alkyl- or alkoxy-groups of 1 to 2 carbon atoms, cycloalcyl- rest of up to 7 carbon atoms or a pyrrol-, pyridine-, piperidine-, pyrimidine-, piperazine- or morpholine-rest which is unsubstituted or substituted at any given position with a methyl-, ethyl- or propyl-group or $R_1$ and $R_2$ are equal or different and represent hydrogen, an alkyl rest with 1 to 4 carbon atoms or a rest of the general formula II

$$—R—Z \qquad (II)$$

in which R is an alkyl group of 1 to 6 carbon atoms or a phenylene group and Z is a hydroxy- and/or alkoxi-group with 1 to 3 carbon atoms which may be at any given position or an amino group of the general formula III

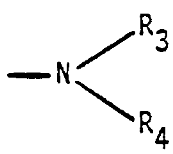

(III)

in which $R_3$ and $R_4$ are equal or different and represent hydrogen or an alkylrest with up to 7 carbon atoms which may be unsubstituted or substituted at any given position or in which $R_2$ is hydrogen or an alkyl rest with up to 7 carbon atoms which may be unsubstituted or substituted at any given position and $R_4$ is a phenyl rest which is unsubstituted or substituted at one or more given positions with one or more amino-, methylamino-, dimethylamino-, hydroxi-, alkyl- or alkoxy-groups with 1 to 2 carbon atoms or a pyrrol-, pyridine-, piperidine, pyrimidine-, piperazine- or morpholine-rest which is unsubstituted or substituted an any given position with a methyl-, ethyl- or propyl-group and X and 4 are equal or different and represent either hydrogen, an alkyl- or alkoxy-group with 1 to 3 carbon atoms or an amino-group of the general formula III.

2. A composition for the dyeing of hair on the basis of oxidation dyestuffs in combination with known developers characterized in that it contains 2-morpholino-5-nitro-pyridine as coupling agent.

3. A composition for a dyeing of hair on the basis of oxidation dyestuffs in combination with known developers characterized in that it contains 2-benzylamino-5-nitropyridine as coupling agent.

**Revendications**

1. Agent pour la teinture des cheveux à base de colorants d'oxydation en combinaison avec des révélateurs appropriés, caractérisé en ce qu'il contient en tant que composant de nuançage un ou plusieurs dérivés de nitropyridine de formule générale I

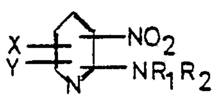

(I)

dans laquelle soit $R_1$ représente l'hydrogène ou un radical alcoyle avec 1-4 atomes de C et $R_2$ représente un radical phényle non substitué ou substitué en n'importe quelle position par un ou plusieurs groupes amino, méthylamino, diméthylamino, hydroxy, alcoyle ou alcoxy avec 1-2 atomes de C, un radical cycloalcoyle avec jusqu'à 7 atomes de C ou un radical pyrrole, pyridine, pipéridine, pyrimidine, pipérazine ou morpholine non substitué ou méthyl-, éthyl-, ou propyl-substitué en une position quelconque, soit $R_1$ et $R_2$ sont identiques ou différents et représentent l'hydrogène, un radical alcoyle avec 1-4 atomes de C ou un radical de formule générale II

$$—R—Z \qquad (II)$$

dans laquelle R représente une chaîne alcoylène de 1-6 atomes de C ou un groupe phénylène et Z représente un groupe hydroxyle et/ou un groupe alcoxy avec 1-3 atomes de C en position quelconque ou un groupe amino de formule générale III

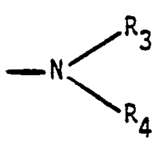

(III)

dans laquelle $R_3$ et $R_4$ sont identiques ou différents l'un de l'autre et représentent l'hydrogène ou un radical alcoyle avec jusqu'à 7 atomes de C non substitué ou hydroxyl- ou amino-substitué en une position quelconque ou $R_3$ représente l'hydrogène ou un radical alcoyle avec jusqu'à 7 atomes de C, non substitué ou hydroxyl- ou amino-substitué en une position quelconque et $R_4$ représente un radical phényle non substitué ou substitué en une position quelconque par un ou plusieurs groupes amino, méthylamino, diméthylamino, hydroxy, alcoyle, ou alcoxy avec 1-2 atomes de C ou représente un radical pyrrole, pyridine, pipéridine, pyrimidine, pipérazine ou morpholine non substitué ou méthyl-, éthyl- ou propyl-substitué en une position quelconque et X et Y sont identiques ou différents l'un de l'autre et représentent chacun soit l'hydrogène, un groupe alcoyle ou un groupe alcoxy avec 1-3 atomes de C, soit un groupe amino de formule générale III.

2. Agent pour la teinture des cheveux à base de colorants d'oxydation en combinaison avec des révélateurs appropriés, caractérisé en ce qu'il contient en tant que composant de nuançage la 2-morpholino-5-nitro-pyridine.

3. Agent pour la teinture des cheveux à base de colorants d'oxydation en combinaison avec des révélateurs appropriés, caractérisé en ce qu'il contient en tant que composant de nuançage la 2-benzylamino-5-nitro-pyridine.